# EUROPEAN PATENT APPLICATION

(11) **EP 3 967 753 A2**
(43) Date of publication of application: **16.03.2022**
(21) Application number: 20769957.0
(22) Date of filing: 05.03.2020
(51) Int. Cl.: C12N 5/0786, C12N 5/0783, A61K 8/98, A61K 35/15, A61K 35/17, A61P 17/00, A61P 17/02, A61P 37/00, A61Q 19/00

(54) **METHOD FOR CULTURING ALLOGENEIC IMMUNE CELL, IMMUNE CELL CULTURE OBTAINED THEREBY, AND IMMUNE CELL THERAPEUTIC AGENT COMPRISING SAME**

(30) Priority: 08.03.2019 KR 20190027152
(71) Applicant: Shin, Ji Seop, Seoul 04355 (KR); Shin, Woo Seop, Gyeonggi-do 13527 (KR)
(72) Inventor: JANG, Min Ji, Seoul 04355 (KR); SHIN, Dong Hyuk, Seongnam-si, Gyeonggi-do 13527 (KR); SHIN, Ji Seop, Seoul 04355 (KR); SHIN, Woo Seop, Seongnam-si, Gyeonggi-do 13527 (KR)
(74) Representative: Beck & Rössig European Patent Attorneys
(86) International application number: PCT/KR2020/095016
(87) International publication number: WO 2020/185056

(57) **Abstract**

The present disclosure relates to a method of culturing natural killer cells (NK cells) applied to immunotherapy. More particularly, the present disclosure relates to a method of culturing an allogeneic immune cell that efficiently amplifies and activates NK cells, which are effective in treating malignant tumors, by culturing lymphocytes derived from the blood of healthy donors rather than patients to whom an immune cell therapeutic agent is to be administered, to an immune cell culture medium obtained using the method, and to an immune cell therapeutic agent including the same.

## Description

### Technical Field

The present disclosure relates to a method of culturing natural killer cells (NK cells) applied to immunotherapy. More particularly, the present disclosure relates to a method of culturing an allogeneic immune cell that efficiently amplifies and activates NK cells, which are effective in treating malignant tumors, by culturing lymphocytes derived from the blood of healthy donors rather than patients to whom an immune cell therapeutic agent is to be administered, to an immune cell culture medium obtained using the method, and to an immune cell therapeutic agent including the same.

### Background Art

NK cells are a kind of lymphocyte and are more specifically large granular lymphocytes. NK cells have the ability to effectively kill infected viruses and tumor cells but do not kill most normal cells. The antitumor action thereof is achieved through the occurrence of necrosis, apoptosis, or both of the action mechanisms. NK cells respond to cytokines such as IL-2, IL-12, and interferon, thereby increasing cytotoxicity, secretory, and proliferative functions. The phenotype of NK cells is CD16 (FcγRIII) or CD56 in humans, and CD16 and CD56 are used as markers for NK cells because there is no T-cell receptor complex (TRC) on the cell surface.

Such NK cells are known to play an important role in the initial biological defense mechanism and tumor immunity of the human body. That is, NK cells may kill specific autologous cells, homogeneous cells, and even heterogeneous cancer cells without the process of acquiring immunity according to the expression of a major histocompatibility complex (MHC). In particular, NK cells are better able to kill target cells that express little or no Class1 MHC. Therefore, NK cells may effectively kill most cancer cells that do not express MHC, and also kill some virus-infected cells and bacteria such as salmonella typhi. However, NK cells, which have such an excellent effect on killing cancer cells, account for only 5 to 15% of peripheral blood lymphocytes even in normal humans. In particular, since the ratio or cytotoxicity thereof is greatly reduced in the case of cancer patients, there is a limit in effectively attacking cancer cells without a separate amplification process through immunotherapy.

Such immunotherapy is a method that includes extracting the most important immune cells to treat cancer, such as natural killer cells (NK cells), dendritic cells (DC), B cells, and T cells, from the blood of a patient, performing growth into an immune cell that strongly acts on cancer using several types of stimulants, and injecting the resultant immune cell back into the patient. Since the patient's own blood is used, there are fewer side effects and the administration method is simple compared to typical chemotherapy, so this method is being actively studied in recent years.

In the case of an anticancer immune cell therapeutic agent, an autoimmune cell therapeutic agent that is used to perform treatment by isolating and culturing immune cells from one's own blood is a significant agent. However, in the case of patients with severe terminal cancer, the number of cells is small and the activity thereof is very small, so cell culturing is sometimes difficult and there are many difficulties such as having to wait for the culturing period. Therefore, if it can be treated by culturing the immune cells of other healthy people, it can be effectively used for patients with severe and terminal cancer, which is not easy to undergo cell culturing.

That is, when such an allogeneic immune cell therapeutic agent is developed, a large amount of immune cells of healthy people may be cultured in advance and frozen to be stored and then administered directly to a patient in a short time when the patient needs, thereby increasing the effect thereof. In order to use other people's immune cells, pure NK cells must be used because other people's Tc cells attack the patient's body. In a typical method of manufacturing pure NK cells, a culturing process is very complicated and expensive because feeder cells such as cell lines undergone tumorogenesis are used or gamma-ray treatment needs to be performed.

Therefore, there is a need for a technology for developing an allogeneic immune cell therapeutic agent from which these drawbacks are eliminated.

### Disclosure

### Technical Problem

The present inventors have made research and efforts to solve the above problems, and as a result, the present inventors have developed a method of culturing an immune cell, causing immunological tolerance to a patient who is to receive T cells of a healthy donor and thus leading changing into cells that are not aggressive, thereby accomplishing the present disclosure.

Accordingly, an objective of the present disclosure is to provide a method of culturing an allogeneic immune cell, in which immunological tolerance occurs in T cells of a healthy person to a patient to whom the immune cell is to be administered, thus changing the T cells into cells that are not aggressive, improving cell proliferation, and prolonging a cell lifespan, an immune cell culture medium obtained using the method, and an immune cell therapeutic agent including the same.

Another objective of the present disclosure is to provide a method of culturing an allogeneic immune cell, in which when immune cells from lymphocyte peripheral blood mononuclear cells (PBMC) isolated from the blood of a healthy donor are cultured, the immune cells are stimulated even without the use of anti-CD3 antibody, so that the culturing is favorably performed and immunological tolerance is induced, thus making the immune cells of others, which are mixed with T cells, available for use in immunotherapy, an immune cell culture medium obtained using the method, and an immune cell therapeutic agent including the same.

The objectives of the present disclosure are not limited only to the objectives mentioned above, and even if not explicitly mentioned, the objective of the disclosure that can be recognized by a person of ordinary skill in the art from the detailed description of the disclosure to be described later may be naturally included therein.

### Technical Solution

In order to accomplish the above-described objectives of the present disclosure, the present disclosure provides a method of culturing an allogeneic immune cell. The method includes culturing a peripheral blood mononuclear cell (PBMC) isolated from a blood of a healthy donor for 9 to 12 days, thus obtaining a first donor cell culture medium, preparing a patient-derived PBMC from a blood of a patient to whom an immune cell therapeutic agent is to be administered, adding the patient-derived PBMC to the first donor cell culture medium, thus obtaining a first mixed culture medium, and a mix-culturing step of culturing the first mixed culture medium.

Further, the present disclosure provides a method of culturing an allogeneic immune cell. The method includes culturing a peripheral blood mononuclear cell (PBMC) isolated from a blood of a healthy donor for 1 to 2 days, thus obtaining a second donor cell culture medium, preparing a patient-derived PBMC from a blood of a patient to whom an immune cell therapeutic agent is to be administered, adding the patient-derived PBMC to the second donor cell culture medium, thus obtaining a second mixed culture medium, and a mix-culturing step of culturing the second mixed culture medium.

In a preferred embodiment, the patient-derived PBMC is any one of a PBMC that is isolated from the blood of the patient and is not cultured, a patient cell culture medium obtained by culturing a PBMC that is isolated from the blood of the patient for 1 to 2 days, and a patient cell culture medium obtained by culturing a PBMC that is isolated from the blood of the patient for 7 to 14 days.

In a preferred embodiment, the first mixed culture medium or the second mixed culture medium is included so that the number of immune cells included in the patient-derived PBMC per 100 immune cells included in the first donor cell culture medium or the second donor cell culture medium is 30 to 200.

In a preferred embodiment, the mix-culturing step is performed for 30 days or more while a step of replacing and harvesting a plurality of medium compositions is performed.

In a preferred embodiment, the step of replacing and harvesting the plurality of medium compositions is performed while the concentration of cytokine is changed at intervals of 3 to 4 days.

Further, the present disclosure provides an allogeneic immune cell culture substance including a medium composition obtained by performing culturing using the above-described any one culturing method, and proliferated immune cells.

Further, the present disclosure provides a culture medium composition in which the proliferated immune cells are removed from the allogeneic immune cell culture substance.

Further, the present disclosure provides an immune cell therapeutic agent including an immune cell obtained after removing a medium composition from the allogeneic immune cell culture substance as an active ingredient.

In a preferred embodiment, the immune cell includes an NK cell, and a T cell that has immunological tolerance to a specific patient to whom the immune cell therapeutic agent is to be administered.

In a preferred embodiment, the T cell that has the immunological tolerance includes an NKT cell, a helper T cell (Th), a cytotoxic T cell (Tc), and a gamma delta T cell.

In a preferred embodiment, the immunological tolerance is obtained by culturing a peripheral blood mononuclear cell (PBMC) isolated from the blood of a healthy donor and also culturing a PBMC derived from a specific patient for a predetermined time.

Further, the present disclosure provides a medium composition obtained in a step of replacing and harvesting a plurality of medium compositions cultured using the method of culturing the allogeneic immune cell.

Further, the present disclosure provides a cosmetic composition including the medium composition as an active ingredient.

Further, the present disclosure provides a pharmaceutical composition for treating injuries or skin diseases including burns and wounds, the pharmaceutical composition including the medium composition as an active ingredient.

### Advantageous Effects

According to the method of culturing the allogeneic immune cell, the immune cell culture medium obtained using the method, and the immune cell therapeutic agent including the same according to the present disclosure, immunological tolerance occurs in T cells of a healthy person to a patient to whom the immune cell is to be administered, thus changing the T cells into cells that are not aggressive, improving cell proliferation, and prolonging a cell lifespan.

Further, in the case of the method of culturing the allogeneic immune cell, the immune cell culture medium obtained using the method, and the immune cell therapeutic agent including the same according to the present disclosure, when immune cells from lymphocyte peripheral blood mononuclear cells (PBMC) isolated from the blood of a healthy donor are cultured, the immune cells are stimulated even without the use of anti-CD3 antibody, so that the culturing is favorably performed and immunological tolerance is induced, thus making the immune cells of others, which are mixed with T cells, available for use in immunotherapy.

These technical effects of the present disclosure are not limited only to the range mentioned above, and even if not explicitly mentioned, the effect of the disclosure that can be recognized by a person of ordinary skill in the art from the description of the specific content for carrying out the disclosure to be described later is naturally included therein.

### Description of Drawings

FIG. 1 is a graph showing FACS data of effector cells used in an experiment to confirm the cell killing ability reduced after inducing the immunological tolerance of T cells;
FIG. 2 is a graph showing the results of the cytotoxicity test of immune cells performed using a typical method to confirm the cell killing ability reduced after inducing the immunological tolerance of T cells;
FIG. 3 is a graph showing the change in the number of cells over time in a method of culturing an allogeneic immune cell according to an embodiment of the present disclosure;
FIGS. 4A to 4C are graphs showing FACS data measured on days 20, 31 and 54 in a method of culturing an allogeneic immune cell according to another embodiment of the present disclosure;
FIG. 5 is a graph showing the change in the number of cells over time in a method of culturing an allogeneic immune cell according to another embodiment of the present disclosure; and
FIG. 6 is a graph showing FACS data measured on 14 days after culturing in FIG. 5.

### Best Mode

The terms used in the present disclosure are only used to describe specific embodiments, and are not intended to limit the present disclosure. The singular expression includes the plural expression unless the context clearly dictates otherwise. In the present application, it should be understood that terms such as "include" or "have" are intended to designate that a feature, number, step, operation, component, part, or combination thereof described in the specification is present, but this does not preclude the possibility of presence or addition of one or more other features, numbers, steps, operations, components, parts, or combinations thereof.

Terms such as first and second may be used to describe various components, but the components should not be limited by the terms. The above terms are used only for the purpose of distinguishing one component from another component. For example, without departing from the scope of the present disclosure, a first component may be referred to as a second component, and similarly, the second component may also be referred to as a first component.

Unless defined otherwise, all terms used herein, including technical or scientific terms, have the same meaning as commonly understood by one of ordinary skill in the art to which the present disclosure belongs. Terms such as those defined in a commonly used dictionary should be interpreted as having a meaning consistent with the meaning in the context of the related art, and do not be interpreted as an ideal or excessively formal meaning unless explicitly defined in the present disclosure.

In the present disclosure, "immune cell" is used in the sense of including only NK cells and T cells. Further, "NK cell" is used in the sense of including both NK cells and NKT cells, but may be interpreted to mean only NK cells in some cases.

In a description of a time relationship, for example, in the case that the temporal front and rear relationship is explained by 'after~', 'following~', 'subsequent to~, or 'before~', it includes cases that are not continuous unless 'immediately' or 'directly' is used.

Hereinafter, the technical constitution of the present disclosure will be described in detail with reference to the accompanying drawings and preferred embodiments.

However, the present disclosure is not limited to the embodiments described herein and may be embodied in other forms. Throughout the specification, like reference numbers used to describe the present disclosure refer to like components.

The technical characteristics of the present disclosure are a method of culturing an allogeneic immune cell, in which even when two types of immune cells of different origins, that is, the immune cell derived from the blood of a healthy donor and the immune cell derived from the blood of a patient, are mixed with each other to perform culturing, immunological tolerance occurs with respect to the cultured T cell, thus changing the T cells into cells that are not aggressive, improving cell proliferation, and prolonging a cell lifespan, an immune cell culture medium obtained using the method, and an immune cell therapeutic agent including the same.

Therefore, the method of culturing the allogeneic immune cell of the present disclosure may be classified into two types as follows depending on the timing of mixing two types of immune cells of different origins and on whether the anti-CD3 antibody is used.

First, a first method of culturing an allogeneic immune cell of the present disclosure may include culturing a peripheral blood mononuclear cell (PBMC) isolated from the blood of a healthy donor for 9 to 12 days, thus obtaining a first donor cell culture medium, preparing a patient-derived PBMC from the blood of a patient to whom an immune cell therapeutic agent is to be administered, adding the patient-derived PBMC to the first donor cell culture medium, thus obtaining a first mixed culture medium, and a mix-culturing step of culturing the first mixed culture medium. Accordingly, the first method of culturing the allogeneic immune cell of the present disclosure has a merit in that since the immune cells of a healthy donor among the two types of immune cells of different origins are cultured for at least 7 days using an anti-CD3 antibody and then are mixed with the patient-derived PBMC, double stimulation is applied to improve the proliferation rate of the immune cells and a lifespan thereof. Further, the first method of culturing the allogeneic immune cell is a method that is effective for a patient having a very low number of NK cells due to an advanced cancer stage, a patient having poor culturing and proliferation of NK cells due to patient specificity, or the case that there are a large number of patients among the donors. In the case of these patients, NK cells are not well cultured, but T cells are relatively well cultured and thus the number of cells may be greatly increased. This is because when cells of different origins are mixed, the cells necessary to provide stimulation may consist only of T cells.

Next, a second method of culturing an allogeneic immune cell of the present disclosure may include culturing a peripheral blood mononuclear cell (PBMC) isolated from the blood of a healthy donor for 1 to 2 days, thus obtaining a second donor cell culture medium, preparing a patient-derived PBMC from the blood of a patient to whom an immune cell therapeutic agent is to be administered, adding the patient-derived PBMC to the second donor cell culture medium, thus obtaining a second mixed culture medium, and a mix-culturing step of culturing the second mixed culture medium. Accordingly, in the second method of culturing the allogeneic immune cell of the present disclosure, by mixing the patient-derived PBMC at the beginning of culturing of peripheral blood mononuclear cells (PBMC) isolated from the blood of a healthy donor at least 3 days before the start of culturing, the donor's immune cells are stimulated even without the use of an anti-CD3 antibody. Therefore, the culturing step becomes simpler and the culturing of the immune cells is favorably performed. Further, the number of immune cells of the donor is small because the culturing is in an early state, so the number of immune cells of the patient needed to induce immunological tolerance may be small.

Both the first mixed culture medium and the second mixed culture medium are included so that the number of immune cells included in the patient-derived PBMC per 100 immune cells included in the donor cell culture medium is 30 to 200. The above mixing ratio is experimentally determined, and the reason why the mixing ratio is set to the above range is that the number of the patient-derived immune cells should be at least 30% or more compared to the number of donor's immune cells in order to induce immunological tolerance and effectively stimulate immune cells for favorable culturing. Meanwhile, as the number of the patient-derived immune cells is increased compared to the number of donor's immune cells, it is easy to induce perfect immunological tolerance. However, the case that the mixing ratio is more than 1:2 may become a burden to critically ill patients, so the maximum mixing ratio was set to 1:2.

Further, the step of preparing the patient-derived PBMC may be performed by preparing any one of a PBMC that is isolated from the blood of the patient and is not cultured, a patient cell culture medium obtained by culturing the isolated PBMC for 1 to 2 days, and a patient cell culture medium obtained by culturing the isolated PBMC for 7 to 14 days. In particular, in the case where the patient is critically ill, when the patient cell culture medium obtained by culturing the patient-derived PBMC for 7 to 14 days is used, proliferation is performed so that the number of cells is large even when a small amount of blood is collected. Accordingly, the patient cell culture medium is capable of being used several times, which is more advantageous. Meanwhile, the patient-derived PBMC may include other monocytes such as B cells in addition to immune cells in a state that is isolated from the blood and is not cultured. However, since only immune cells survive in the subsequent culturing process, the patient cell culture medium may substantially include only immune cells.

Meanwhile, in the first and second methods of culturing the allogeneic immune cell of the present disclosure, since two types of immune cells of different origins are mixed and cultured, the lifespan of immune cells is extended. Accordingly, the mix-culturing step may be performed for 30 days or more while a step of replacing and harvesting a plurality of medium compositions is performed. The step of replacing and harvesting the plurality of medium compositions may be performed while the concentration of cytokine is changed at intervals of 3 to 4 days. Meanwhile, when the lifespan of the cells is extended in this way, it is possible to secure a large amount of a culture medium, that is, a medium composition, from which immune cells containing a large amount of useful components such as cytokines are removed. As is well known, the medium composition containing a large amount of useful components may be a raw material for cosmetics or pharmaceuticals. Therefore, according to the method of culturing the allogeneic immune cell of the present disclosure, it is possible to very economically obtain a medium composition that contains a large amount of useful components but does not contain serum. In particular, when the method of culturing the allogeneic immune cell of the present disclosure is performed in order to obtain a medium composition that is a raw material for cosmetics or pharmaceuticals, performing culturing using PBMCs derived from the blood of two healthy donors rather than those derived from a healthy donor and a patient as two types of immune cells of different origins may be more useful to obtain a large amount of medium composition.

The medium composition thus obtained may be used as an active ingredient in cosmetic compositions and pharmaceutical compositions. In particular, the medium composition of the present inventor is effective for one or more of skin whitening, pigmentation removal, and wrinkle overcoming as described in Korean Laid-Open Patent Application No. 10-2018-0057359, which is a prior patent of the present inventor. The medium composition is effective in overcoming inflammation, such as allergic rhinitis, allergic dermatitis, atopic dermatitis, acne, and inflammation caused by insect bites. In particular, the medium composition is effective in quickly removing itching. Further, it was confirmed that the medium composition has a skin regeneration effect through the treatment of burns and wounds and is very effective in removing deposited pigments and also in skin whitening.

Next, the allogeneic immune cell culture substance of the present disclosure includes the medium compositions obtained by performing the first and second methods of culturing the allogeneic immune cell and the proliferated immune cells. The medium compositions and the proliferated immune cells may be isolated from the allogeneic immune cell culture substance to use the culture medium composition from which the proliferated immune cells are removed as an active ingredient in cosmetic compositions or pharmaceutical compositions and to use the isolated immune cells as an active ingredient in an immune cell therapeutic agent.

Therefore, the immune cell therapeutic agent of the present disclosure includes the immune cells isolated from the allogeneic immune cell culture substance as the active ingredient. The immune cells include NK cells and T cells having immunological tolerance to a specific patient to whom the immune cell therapeutic agent is to be administered. The T cells having immunological tolerance include NKT cells, helper T cells (Th), cytotoxic T cells (Tc), and gamma delta T cells. As described above, the immunological tolerance is obtained by culturing a peripheral blood mononuclear cell (PBMC) isolated from the blood of a healthy donor and also culturing a PBMC derived from a specific patient for a predetermined time. The immune cell therapeutic agent of the present disclosure may be generally administered into the patient's body in a Ringer's type by including immune cells in a carrier such as physiological saline, and does not affect immune cells. However, of course, the immune cell therapeutic agent may further include a known component that is effective in treating patients.

The medium addition kit (NKTM) for culturing immune cells used in the first and second methods of culturing the allogeneic immune cell of the present disclosure described above is individually packaged so as to be added to the medium at each stage of lymphocyte culturing, and includes contents of different constituent components, that is, a B unit, a C1-1 unit, a C1-2 unit, a C2 unit, a A1 unit, a A2 unit, and a D unit. A detailed description of each unit and a detailed description of specific conditions for a culture process (NKTM culturing process) of culturing immune cells using the unit are disclosed in Korean Laid-Open Patent Application No. 10-2018-0057359, which is a prior patent, (hereinafter referred to as "prior patent"), so only the parts different from those described in the prior patent will be mainly described in the following Examples.

### Mode for Disclosure

### Example 1

A first method of culturing an allogeneic immune cell was performed as follows.

### 1. Step of obtaining a first donor cell culture medium

First, the blood of a healthy donor was superimposed on a Ficoll-Paque Plus solution having a specific gravity of 1.077 using the property that mononuclear cells such as human lymphocytes or monocytes have a specific gravity lower than 1.077 to perform centrifugal precipitation with a constant centrifugal force. Thereby, according to the difference in specific gravity, separation was performed so that the erythrocyte and granulocyte layer having a specific gravity of more than 1.077 was positioned on the bottom and the mononuclear cell layer and platelets having a specific gravity of 1.077 or less were positioned on the top, thus obtaining a PBMC including lymphocytes. If necessary, it is possible to extract and use only lymphocytes from the PBMC. However, even when the PBMC is cultured without any modification, the same results can be obtained because the remaining cells except for immune cells (NK cells and T cells) are removed under the subsequent culturing condition. Accordingly, PBMCs and lymphocytes may be analyzed and used in the same way.

Thereafter, the PBMC isolated from the blood of a healthy donor was cultured according to the method described in Examples of the prior patent, that is, the NKTM culturing method, for Day 0 to Day 10, thus obtaining a first donor cell culture medium.

### 2. Step of preparing a patient-derived PBMC

The PBMC, which was isolated from the blood of a patient to whom an immune cell therapeutic agent is to be administered by performing the above-described method, was cultured according to the NKTM culturing method for Day 0 to Day 10, thus obtaining a patient cell culture medium.

### 3. Step of obtaining a first mixed culture medium

Unlike the prior patent in which immune cells and a serum-free primary culture medium were isolated from a primary culture medium including cells through centrifugation (400xg) to collect the serum-free primary culture medium on Day 11, in the present disclosure, the prepared first donor cell culture medium and the patient cell culture medium were mixed at a volume ratio of 1:1 to obtain a first mixed culture medium.

### 4. Mix-culturing step

The first mixed culture medium was cultured for Day 12 to Day 18 according to the NKTM culturing method to obtain an allogeneic immune cell culture substance 1 including a medium composition and proliferated immune cells.

### Example 2

Culturing was performed for 60 days while performing replacement and harvesting of the medium composition in the same manner as in Table 1 below after obtaining the first mixed culture medium in the same manner as in Example 1, except that the donor's blood and the patient's blood were changed, thereby obtaining a large amount of medium composition.

On Day 11, the cells in two bags were mixed with each other and then divided into two bags, followed by culturing. Further, the stimulus was changed while the concentration of cytokine in the culture medium was changed at intervals of 3 to 4 days, and culturing was performed while the medium was replaced. The change in the concentration of cytokine may be implemented in a way that repeats the process of from day 8 to day 14 of the NKTM culture process. That is, after culturing was performed under a high concentration of C1 solution, a culture bag including no cytokine was used to perform culturing, and the concentration of cytokine may be changed by loosening the culture bag little by little. When the culturing is performed in this way, a large amount of immune cell culture medium rich in cytokine may be obtained using a small amount of immune cells.

**[Table 1]**

| Culturing day | Culturing method | Culturing day | Culturing method |
|---|---|---|---|
| 11 | Medium composition replacement, cell mixing | 32 | Medium composition replacement (Medium composition harvesting) |
| 13 | | 34 | |
| 15 | Medium composition replacement (Medium composition harvesting) | 37 | Medium composition replacement (Medium composition harvesting) |
| 18 | Medium composition replacement (Medium composition harvesting) | 39 | |
| 20 | Medium composition replacement (Medium composition harvesting) | 42 | Medium composition replacement (Medium composition harvesting) |
| 22 | Medium composition replacement (Medium composition harvesting) | 44 | |
| 24 | | 49 | Medium composition replacement (Medium composition harvesting) |
| 24 | Medium composition replacement (Medium composition harvesting) | 53 | Medium composition replacement (Medium composition harvesting) |
| 29 | | 54 | |
| 31 | | 60 | |

### Example 3

A second method of culturing an allogeneic immune cell was performed as follows.

### 1. Step of obtaining a second donor cell culture medium

The PBMC isolated from the blood of another donor in the same manner as in Example 1 was cultured according to the NKTM culturing method for Day 0 to Day 1, thereby obtaining a second donor cell culture medium. The number of immune cells was 1.2 × 10⁷.

### 2. Step of preparing a patient-derived PBMC

The PBMC isolated from the blood of the patient to whom the immune cell therapeutic agent is to be administered was cultured for Day 0 to Day 14 according to the NKTM culturing method, thus obtaining a patient cell culture medium.

### 3. Step of obtaining a second mixed culture medium

The patient cell culture medium was added instead of the D unit to be added to the second donor cell culture medium on Day 2 so that the number of immune cells was 0.8 × 10⁷ according to the NKTM culturing method, thus obtaining a second mixed culture medium.

### 4. Mix-culturing step

The second mixed culture medium was cultured according to the NKTM culturing method for Day 2 to Day 14, thus obtaining an allogeneic immune cell culture substance 2 including a medium composition and proliferated immune cells.

### Experimental Example 1

As follows, an experiment was performed to confirm the reduced cell killing ability after inducing immunological tolerance of T cells using basal stem cells. That is, the immunological tolerance of the T cells was induced using stem cells of a specific person, and the killing abilities of T cells against these stem cells were compared, thus confirming the immunological tolerance. The stem cells are known to express less MHC class I molecules on the cell surface compared to other cells. As the stem cells used in the experiment, passage8 stem cells obtained from an umbilical cord were used. The immune cells harvested on day 14 of the cell culturing (NK cell purity: 69%) were used, and stem cells were mixed with these immune cells so that the amount of the stem cells was about 30% and then cultured for one day, followed by a cytotoxicity test of the immune cells. That is, the immune cells were used as effector cells, and human stem cells, such as the stem cells cultured the previous day, were used as target cells to perform the test. The cytotoxicity test of the immune cells was performed using the following typical method.

### [Cytotoxicity test]

The target cells were subjected to fluorescence staining in vitro to be reacted with the effector cells, and the intensity of fluorescence emitted from target cells attacked and thus destroyed by the effector cells was then measured, thereby evaluating the killing ability of the effector cells. The higher the cytotoxic activity, the higher the measured value of fluorescence intensity.

A calcein AM which is a fluorescent reagent is a lipid-soluble diester fluorogenic esterase substrate and may pass through a cell membrane. In living cells, hydrolysis was performed using intracellular esterase of an intact cell membrane to convert the calcein AM (non-fluorescence) into calcein (fluorescence), thereby ensuring fluoresces. The esterase activity is maintained due to the intact membrane of the living cells, thereby ensuring fluorescence. However, in the case of the damaged cell membrane of dead cells, the esterase activity is lost and fluorescence is emitted, so the fluorescence is not ensured (in the case of dead cells, the fluorescence cannot be maintained).

When the target cell is fluorescently stained with the calcein AM and thus reacted with the effector cell, the target cell attacked by the effector cell is destroyed and emits fluorescence, and only the living target cell has fluorescence. As the damage by the effector cell is increased, the intensity of fluorescence emitted from the destroyed target cell is increased. The intensity of fluorescence emitted from the target cell may be measured using a device capable of measuring the intensity of fluorescence.

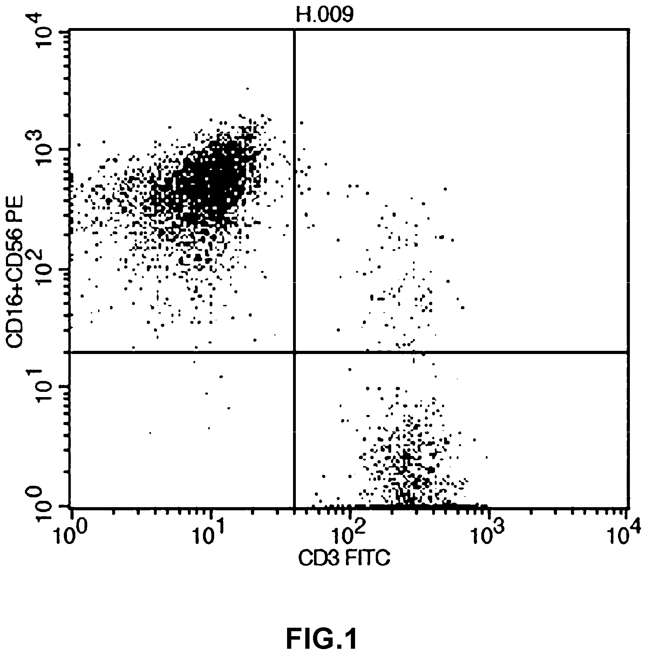

The experiment was performed in the following order.
1) Target cells were collected in a 15 ml tube and centrifuged at 1500 rpm for 5 minutes. The supernatant was removed and the pellet was dissolved with a 1 ml culture medium (RPMI1640 + 10% FBS), followed by cell counting.
2) After the number of cells was set to 1×10⁶/ml in a 1.5 ml tube, 10 µl of a dyeing solution of a calcein AM (1mg/DMSO 1ml) was added, followed by incubation with 5% CO2 at 37°C for 30 minutes.
3) After the incubation, the supernatant was removed, and the cells were washed with 1 ml of a culture medium (RPMI1640 + 10% FBS) (repeated twice).
4) The effector cells were centrifuged at 1500 rpm for 5 minutes, followed by washing once with a culture medium (RPMI1640 + 10% FBS). FIG. 1 shows the FACS data of the effector cell used in the experiment.
5) After the supernatant was removed, 1 ml of a culture medium was added. Then, cell counting was performed with a hemocytometer to prepare the cells so that the cell concentration was 10 times and 5 times larger than the concentration of the target cell.
6) A control and target cells/effector cells were manufactured in a 96 well plate and then dispensed in wells.
7) Natural glass well (negative control): 100 µl of culture medium (RPMI1640 + 10%FBS) + 50 µl of target cell
8) Max glass well (positive control): 70 µl of culture medium (RPMI1640 + 10%FBS) + 50 µl of target cell + 30 µl of 1% triton X-100
9) Culture medium well: 150 µl of culture medium (RPMI1640 + 10% FBS)
10) Sample well: 50 µl of target cell + 100 µl of effector cell
11) The culture medium and the specimen were mixed for good reaction.
12) Incubation was performed with 5% CO2 at 37°C for 4 hours.
13) After the reaction, centrifuging was performed at 1500 rpm for 5 minutes.
14) After the centrifuging, 120 µl of the resultant material was moved to a black plate to measure a fluorescence value with a fluoroskan ascent plate reader.
15) Cytotoxicity was calculated using the following calculation formula.

Cytotoxicity (%) of effector cell = (fluorescence value of sample - natural glass fluorescence value) / (maximum glass fluorescence value - natural glass fluorescence value) × 100

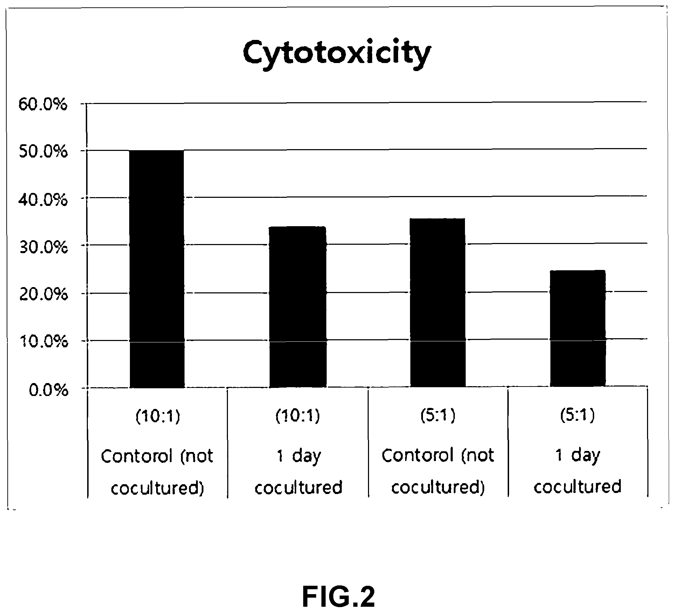

The cytotoxicities of the co-cultured immune cells (effector: target cells) obtained through the above experiment were compared, and the results are shown in Table 2 and FIG. 2.

**[Table 2]**

| | Control (not co-cultured) | 1 day Co-cultured | Control (not co-cultured) | 1 day Co-cultured |
|---|---|---|---|---|
| Effector/target cell | (10:1) | (10:1) | (5:1) | (5:1) |
| Cytotoxicity | 49.7% | 33.8% | 35.5% | 24.3% |

From the results of Table 2 and FIG. 2, it could be confirmed that immunological tolerance occurred. That is, it is known that in the case of stem cells, a lot of MHC class I molecules are not expressed on the surface thereof. In this case, it is easy to be attacked by NK cells, and stem cells in which a lot of MHC class I molecules are expressed are attacked by other people's Tc (CD8 + T cells) due to differences thereof and then died. This is because, as shown in Table 2 and FIG. 2, the activity of immune cells in which the immunological tolerance was induced by mixing and culturing the stem cells was confirmed to be much lower than that of immune cells in which the immunological tolerance was not induced. However, it is thought that the activity is still maintained to some extent due to NK cells, and it was confirmed that immunological tolerance to the target cells was induced by culturing the homogeneous cells together. Through this, it can be seen that it is possible to more easily manufacture the homogeneous immune cell therapeutic agents without removing T cells.

### Experimental Example 2

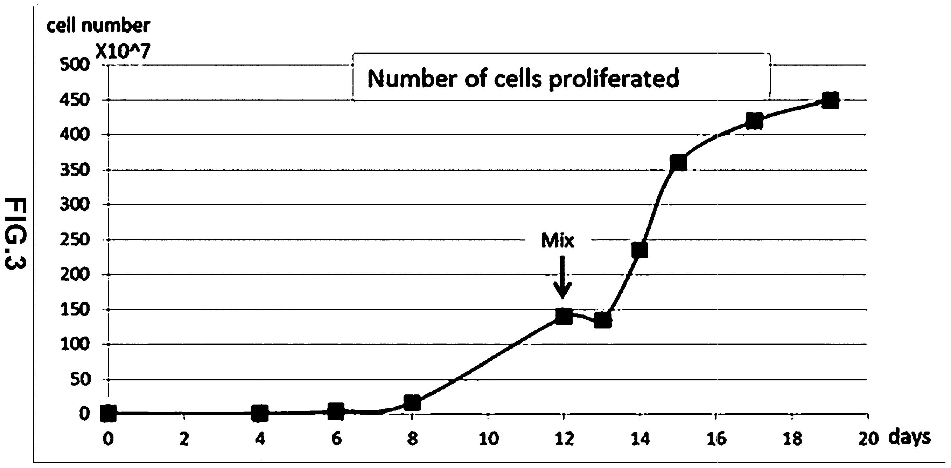

In Example 1, culturing was performed for Day 0 to Day 18, and the number of immune cells according to the culturing day was observed. The results are shown in Table 3 and FIG. 3.

**[Table 3]**

| Culturing day | 0 | 4 | 6 | 8 | 12 | 13 | 14 | 15 | 17 | 19 |
|---|---|---|---|---|---|---|---|---|---|---|
| Number of cells in first donor cell culture medium (x 10⁷) | 0.5 | 0.6 | 2.3 | 8 . | 71.5 | | | | | |
| Number of cells in patient cell culture medium (x 10⁷) | 0.55 | 0.5 | 2.1 | 8.1 | 68.3 | | | | | |
| Total number of cells (x 10⁷) | 1.05 | 1.1 | 4.4 | 16.6 | 139.8 | 135 | 235 | 360 | 420 | 450 |

In the results of Table 3 and FIG. 3, when the cells of the two people are mixed with each other, the Tc cells initially attack each other and thus the number of cells is slightly reduced. However, after the immunological tolerance occurs, the cells of the two people do not attack each other. Further, the immune cells become more activated by stimulation due to differences and thus proliferate more than expected. Further, it can be seen that the cell lifespan is increased and thus cell harvesting is possible between day 14 and day 21.

### Experimental Example 3

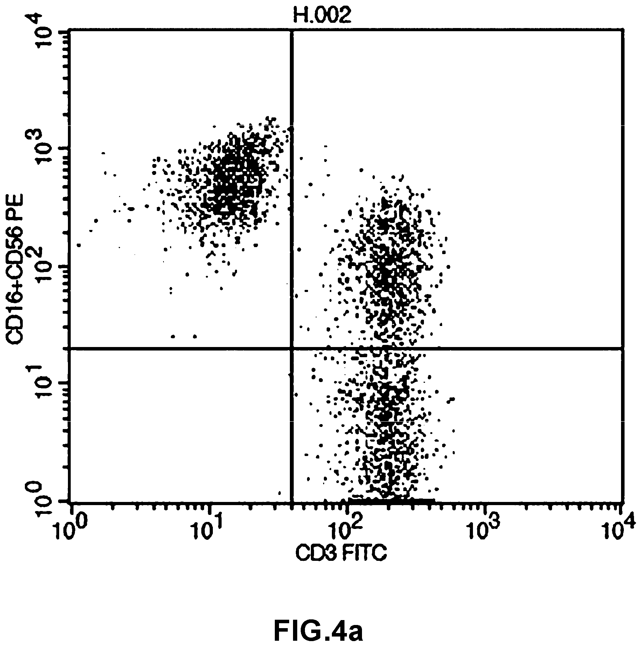

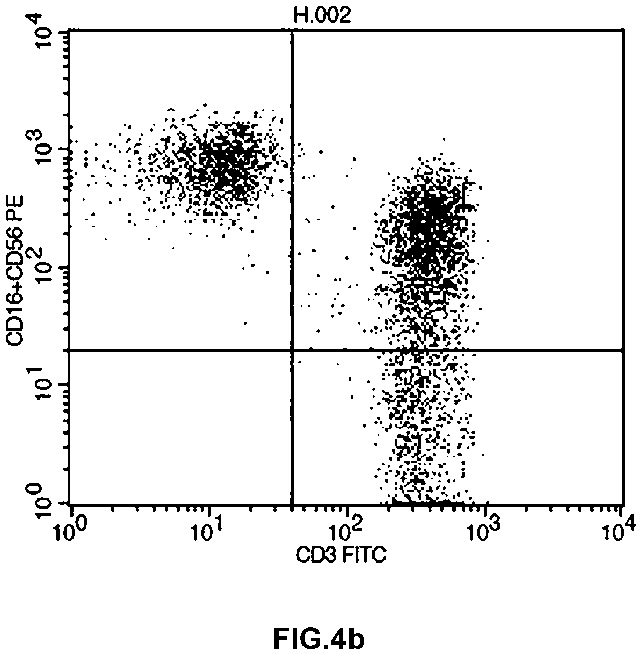

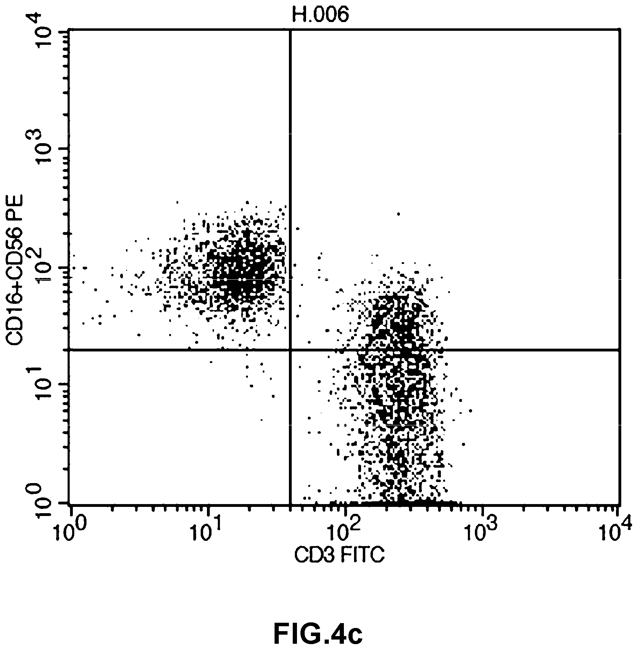

In Example 2, culturing was performed for Day 0 to Day 60, and the number of immune cells according to the culturing day was observed. The results are shown in Table 4 and FIGS. 4A to 4C.

**[Table 4]**

| Culturing day | Number of cells (×10⁷) | | Others |
|---|---|---|---|
| | Second donor cell culture medium | Patient cell culture medium | |
| 0 | 1.1 | 0.9 | |
| 4 | 0.7 | 0.5 | |
| 6 | 3.96 | 2.1 | |
| 8 | 14.1 | 8.5 | |
| 11 | 51.2 | 40.5 | |
| 13 | | | |
| 15 | | | |
| 18 | | | |
| 20 | 305.2 | | NK:35.16, NKT:29.3, gdT:35.12 |
| 22 | 303.5 | | |
| 24 | 350 | | |
| 24 | | | |
| 29 | | | |
| 31 | 389 | | NK:29.13, NKT:50.7, gdT:34 |
| 32 | | | |
| 34 | 420 | | |
| 37 | | | |
| 39 | 363 | | |
| 42 | | | |
| 44 | 303 | | |
| 49 | | | |
| 53 | | | |
| 54 | 215 | NK:35.88, NKT:16.2, gdT:18.52 Cytotoxicity (10:1): 41.2% | |
| 60 | 120 | | |

As can be seen from Table 4 and FIGS. 4A to 4C, when the cells of the two people are mixed with each other in the same manner as in Experimental Example 2, the Tc cells initially attack each other and thus the number of cells is slightly reduced. However, after immunological tolerance occurs, the Tc cells do not attack each other. Further, the immune cells become more activated by stimulation due to differences and thus proliferate more than expected. NK cells also showed very large cytotoxicity to the K562 cell line, so the activity of NK immune cells was found to be maintained regardless of immunological tolerance. Further, it could be confirmed that the cell lifespan was very long. In a typical culturing method, the number of cells was rapidly reduced after about 17 to 20 days. However, when the culturing was performed while other people's cells were mixed, the concentration of cytokine was changed in the culture medium at intervals of 3 to 4 days, the stimulation was changed, and the medium was replaced, a large number of immune cells was confirmed to be viable even if the lifespan thereof was 60 days or more.

### Experimental Example 4

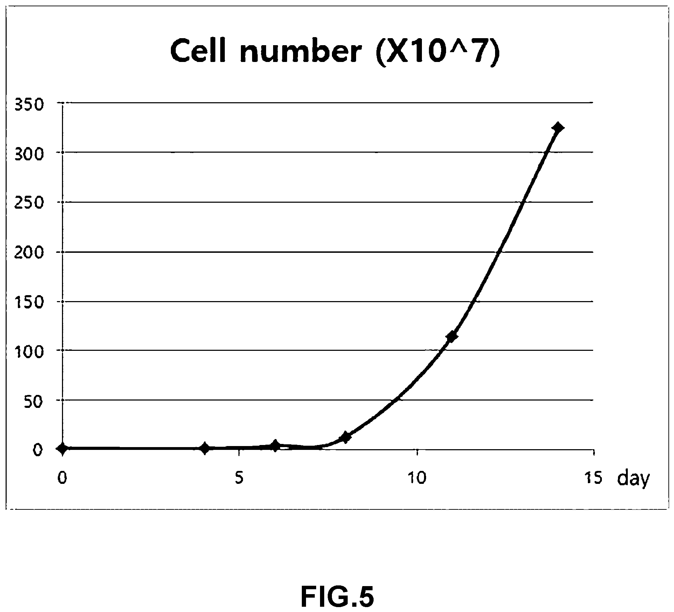

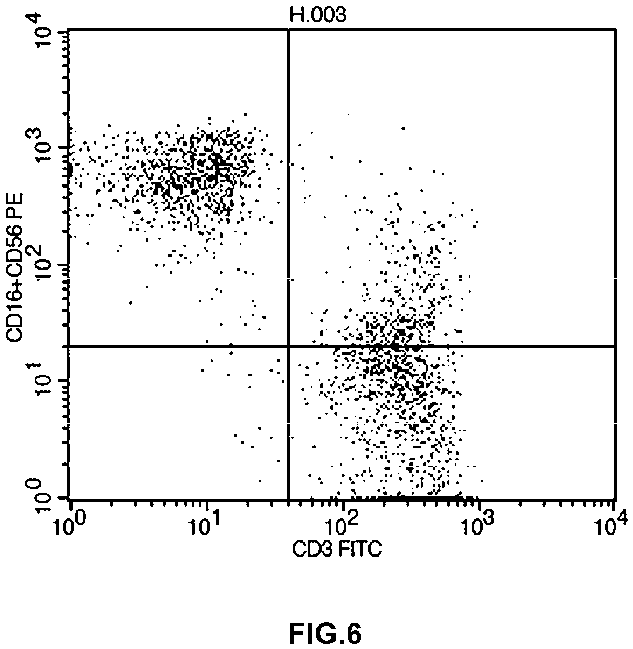

In Example 3, culturing was performed for Day 0 to Day 14, and the number of immune cells according to the culturing day was observed. The results are shown in Table 5 and FIGS. 5 and 6.

**[Table 5]**

| Culturing day | 0 | 4 | 6 | 8 | 11 | 14 |
|---|---|---|---|---|---|---|
| Number of cells (×10⁷) | 1.2 | 1.0 | 3.6 | 13.1 | 115 | 325 |

In Table 5 and FIGS. 5 and 6, when the cells of the two people are mixed with each other, the Tc cells initially attack each other and thus the number of cells is slightly reduced. However, after immunological tolerance occurs, the Tc cells do not attack each other. Even without stimulation using an anti-CD3 antibody at the initial stage of culturing according to the second method of culturing the allogeneic immune cell of the present disclosure, it can be seen that the immune cells become more activated by stimulation due to the immune cells of patients of different origins, that is, due to differences, and thus proliferate more than expected.

The present disclosure has been illustrated and described with reference to preferred embodiments as described above. However, the present disclosure is not limited to the above embodiments, and various changes and modifications can be made by those skilled in the art to which the present disclosure pertains within the scope of not departing from the spirit of the present disclosure.

## Claims

1. A method of culturing an allogeneic immune cell, the method comprising:
culturing a peripheral blood mononuclear cell (PBMC) isolated from a blood of a healthy donor for 9 to 12 days, thus obtaining a first donor cell culture medium;
preparing a patient-derived PBMC from a blood of a patient to whom an immune cell therapeutic agent is to be administered;
adding the patient-derived PBMC to the first donor cell culture medium, thus obtaining a first mixed culture medium; and
a mix-culturing step of culturing the first mixed culture medium.

2. A method of culturing an allogeneic immune cell, the method comprising:
culturing a peripheral blood mononuclear cell (PBMC) isolated from a blood of a healthy donor for 1 to 2 days, thus obtaining a second donor cell culture medium;
preparing a patient-derived PBMC from a blood of a patient to whom an immune cell therapeutic agent is to be administered;
adding the patient-derived PBMC to the second donor cell culture medium, thus obtaining a second mixed culture medium; and
a mix-culturing step of culturing the second mixed culture medium.

3. The method of claim 1 or 2, wherein the patient-derived PBMC is a PBMC that is isolated from the blood of the patient and is not cultured, or a patient cell culture medium obtained by culturing a PBMC that is isolated from the blood of the patient for 7 to 14 days.

4. The method of claim 1 or 2, wherein the first mixed culture medium or the second mixed culture medium is included so that a number of immune cells included in the patient-derived PBMC per 100 immune cells included in the first donor cell culture medium or the second donor cell culture medium is 30 to 200.

5. The method of claim 1 or 2, wherein the mix-culturing step is performed for 30 days or more while a step of replacing and harvesting a plurality of medium compositions is performed.

6. The method of claim 5, wherein the step of replacing and harvesting the plurality of medium compositions is performed while a concentration of cytokine is changed at intervals of 3 to 4 days.

7. An allogeneic immune cell culture substance comprising:
a medium composition obtained by performing culturing using the culturing method of claim 1 or 2; and
proliferated immune cells.

8. A culture medium composition in which proliferated immune cells are removed from the allogeneic immune cell culture substance of claim 7.

9. An immune cell therapeutic agent comprising:
an immune cell obtained after removing a medium composition from the allogeneic immune cell culture substance of claim 7 as an active ingredient.

10. The immune cell therapeutic agent of claim 9, wherein the immune cell includes an NK cell, and a T cell that has immunological tolerance to a specific patient to whom the immune cell therapeutic agent is to be administered.

11. The immune cell therapeutic agent of claim 10, wherein the T cell that has the immunological tolerance includes any one or more among an NKT cell, a helper T cell (Th), a cytotoxic T cell (Tc), and a gamma delta T cell.

12. The immune cell therapeutic agent of claim 11, wherein the immunological tolerance is obtained by culturing a peripheral blood mononuclear cell (PBMC) isolated from a blood of a healthy donor and also culturing together with a PBMC derived from a specific patient for a predetermined time.

13. A medium composition obtained in a step of replacing and harvesting a plurality of medium compositions cultured using the method of culturing the allogeneic immune cell of claim 5.

14. A cosmetic composition comprising:
the medium composition of claim 13 as an active ingredient.

15. A pharmaceutical composition for treating injuries or skin diseases including burns and wounds, the pharmaceutical composition comprising:
the medium composition of claim 13 as an active ingredient.
